# EUROPEAN PATENT APPLICATION

(11) **EP 3 751 394 A1**
(43) Date of publication of application: **16.12.2020**
(21) Application number: 18904972.9
(22) Date of filing: 14.11.2018
(51) Int. Cl.: G06F 3/01

(54) **CONTROL DEVICE, CONTROL METHOD, AND PROGRAM**

(30) Priority: 09.02.2018 JP 2018021611
(71) Applicant: Sony Corporation, 108-0075 Tokyo (JP)
(72) Inventor: IRIE, Atsushi, Tokyo 108-0075 (JP); HANDA, Masaki, Tokyo 108-0075 (JP); AKAMA, Taketo, Tokyo 108-0075 (JP); HO, Hsingying, Tokyo 108-0075 (JP); OHASHI, Takeshi, Tokyo 108-0075 (JP); KAWANO, Midori, Tokyo 108-0075 (JP)
(74) Representative: MFG Patentanwälte Meyer-Wildhagen Meggle-Freund Gerhard PartG mbB
(86) International application number: PCT/JP2018/042051
(87) International publication number: WO 2019/155710

(57) **Abstract**

Provided is a control device including a control unit that outputs information that controls output of scent on the basis of a result of recognizing a hand.

## Description

### TECHNICAL FIELD

The present disclosure relates to a control device, a control method, and a program.

### BACKGROUND ART

A device that converts information based on perception (for example, smell) of a user into information based on perception of another animal and provides the user with the converted information is known (refer to Patent Document 1 below, for example).

### CITATION LIST

### PATENT DOCUMENT

Patent Document 1: Japanese Patent Application Laid-Open No. 2014-165706

### SUMMARY OF THE INVENTION

### PROBLEMS TO BE SOLVED BY THE INVENTION

In such a field, it is desired that information regarding perception be provided to the user on the basis of appropriate information.

An object of the present disclosure is to provide, for example, a control device, a control method, and a program that output information that controls output of scent (smell) on the basis of appropriate information.

### SOLUTIONS TO PROBLEMS

The present disclosure is, for example,
a control device including
a control unit that outputs information that controls output of scent on the basis of a result of recognizing a hand.

The present disclosure is, for example,
a control method including
outputting, by a control unit, information that controls output of scent on the basis of a result of recognizing a hand.

The present disclosure is, for example,
a program that causes a computer to execute a control method including
outputting, by a control unit, information that controls output of scent on the basis of a result of recognizing a hand.

### EFFECTS OF THE INVENTION

According to at least one embodiment of the present disclosure, information that controls output of scent (smell) can be output on the basis of appropriate information. The effects described here are not necessarily limited, and may be any of the effects described in the present disclosure. Furthermore, the content of the present disclosure is not to be interpreted as being limited by the exemplified effects.

### BRIEF DESCRIPTION OF DRAWINGS

Fig. 1 is a block diagram illustrating a configuration example of a control system according to an embodiment.
Fig. 2 is a diagram illustrating an exemplary appearance of the control device according to the embodiment.
Fig. 3 is a block diagram illustrating a configuration example of the control device according to the embodiment.
Fig. 4 is a block diagram illustrating a configuration example of a scent output device according to the embodiment.
Figs. 5A to 5D are diagrams illustrating specific examples of the scent output device according to the embodiment.
Fig. 6 is a diagram for schematically describing processing to recognize a hand motion, or the like.
Fig. 7 is a diagram for describing an example of processing to estimate a hand position.
Fig. 8 is a diagram for describing an example of processing to estimate a hand position.
Fig. 9 is a diagram for describing an example of processing to estimate a hand position.
Figs. 10A to 10D are diagrams for describing an overview of processing according to a first embodiment.
Fig. 11 is a flowchart for describing a flow of processing according to the first embodiment.
Fig. 12 is a flowchart for describing a flow of processing (modification) according to the first embodiment.
Figs. 13A to 13D are diagrams for describing an overview of processing according to a second embodiment.
Fig. 14 is a flowchart for describing a flow of processing according to the second embodiment.
Fig. 15 is a flowchart for describing a flow of processing (modification) according to the second embodiment.
Fig. 16 is a flowchart for describing a flow of processing (modification) according to the second embodiment.
Fig. 17 is a flowchart for describing a flow of processing (modification) according to the second embodiment.
Figs. 18A to 18D are diagrams for describing an overview of processing according to a third embodiment.
Fig. 19 is a flowchart for describing a flow of processing according to the third embodiment.

### MODES FOR CARRYING OUT THE INVENTION

Embodiments and the like of the present disclosure will be described below with reference to the drawings. Note that the description will be made in the following order.

### <About technology common to embodiments>

### <First embodiment>

### <Second embodiment>

### <Third embodiment>

### <Modifications>

### <About technology common to embodiments>

### [About control system]

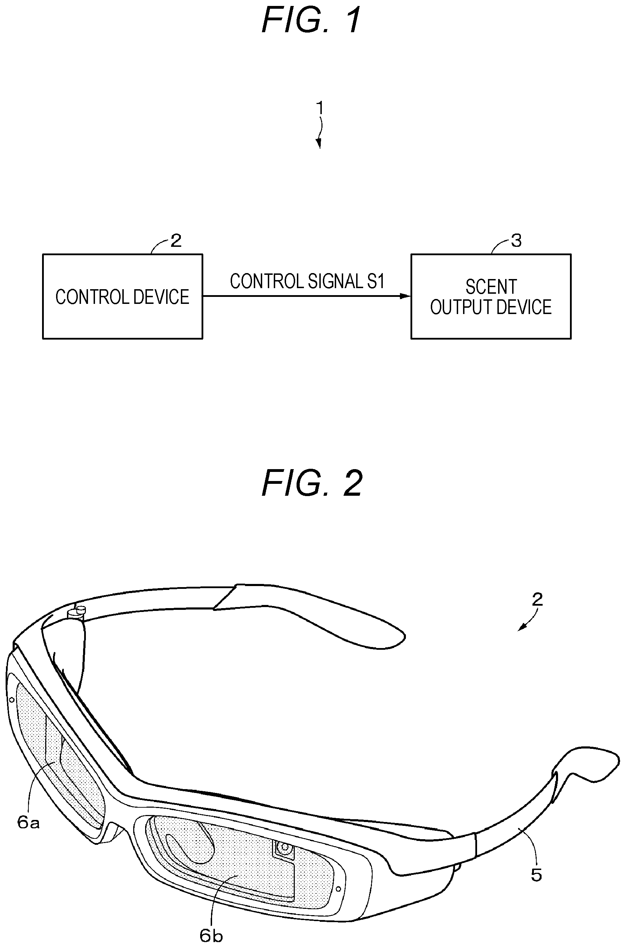

First, technology common to each embodiment of the present disclosure will be described. Fig. 1 is a diagram illustrating a configuration example of a control system (control system 1) according to embodiments of the present disclosure. The control system 1 has a configuration that includes, for example, a control device 2 and a scent output device 3.

Operation on the control system 1 will be schematically described. The control device 2 outputs information that controls output of scent (smell) on the basis of a result of recognizing a hand. The control device 2 outputs a control signal S1 that is information that controls output of scent on the basis of, for example, a recognition result of a shape of a hand of a user or a recognition result regarding continuous change in shape of a hand, in other words, a hand position or hand motion. The control signal S1 output from the control device 2 is supplied to the scent output device 3. The scent output device 3 emits, or the like, scent according to the control signal S1. Note that the scent output device 3 may be capable of emitting, or the like, a plurality of types of scent according to an object.

Note that output of scent means controlling scent, and, specifically, the meaning includes at least one of causing scent to be emitted (released), causing intensity of scent to be changed, causing a direction in which scent comes to be changed, canceling emission of scent, or time duration for which scent emission lasts. Therefore, there may be a case where the scent output device 3 emits scent, stops emitting scent, changes intensity of scent, or the like, according to the control signal S1.

In the control system 1, there may be one or a plurality of scent output devices 3. Furthermore, although the scent output device 3 is described as a device physically separated from the control device 2 in the embodiments, the scent output device 3 may be a device physically integrated to the control device 2, in other words, the control device 2 may have the scent output device 3. Moreover, the scent output device 3 may be a device detachable from the control device 2.

The control system 1 may include a device other than the control device 2 and the scent output device 3. For example, the control system 1 may have a device that causes the user to perceive a change in surrounding environment (hereinafter referred to as an environmental change emission device, as appropriate). Examples of such an environmental change emission device include a device that emits sound (for example, a portable speaker device), a device that causes a fan, or the like, to emit wind, a lighting device, and the like. There may be one or a plurality of environmental change emission devices. Furthermore, different types of environmental change emission devices may be used. Operation of an environmental change emission device is controlled on the basis of a control signal S2, which is information that causes the user to perceive a change in surrounding environment. The control signal S2 is, for example, supplied from the control device 2 to the environmental change emission device. Such an environmental change emission device may be incorporated in the control device 2 or the scent output device 3.

### [About control device]

### (Exemplary appearance)

Next, the control device 2 will be described. Fig. 2 is a diagram illustrating an exemplary appearance of the control device 2. The control device 2 is, for example, configured as a wearable apparatus (an apparatus that can be worn on a human body and has a portable size). More specifically, the control device 2 according to the embodiments is configured as a glasses-type terminal device as illustrated in Fig. 2.

The control device 2 has a frame 5 for retaining a right image display unit 6a and a left image display unit 6b, as similar to ordinary glasses. The right image display unit 6a and the left image display unit 6b are arranged so as to be positioned in front of a right eye and left eye of the user, respectively.

The frame 5 is provided with a sensor of various kinds, an imaging device, a battery, or the like (illustration of these are omitted). On the right image display unit 6a and the left image display unit 6b, for example, an image (actual image) obtained via the imaging device is displayed. By providing the imaging device on an appropriate position of the frame 5, an image from a viewpoint of the user can be displayed on the right image display unit 6a and the left image display unit 6b.

Instead of an actual image, an image generated by the control device 2 or an image supplied from an external device may be displayed on the right image display unit 6a and the left image display unit 6b. In this case, space, so-called virtual reality (VR), can be provided to the user. Furthermore, an actual image to which an image of a predetermined object is added may be displayed on the right image display unit 6a and the left image display unit 6b. In this case, space, so-called augmented reality (AR), can be provided to the user.

### (Configuration example)

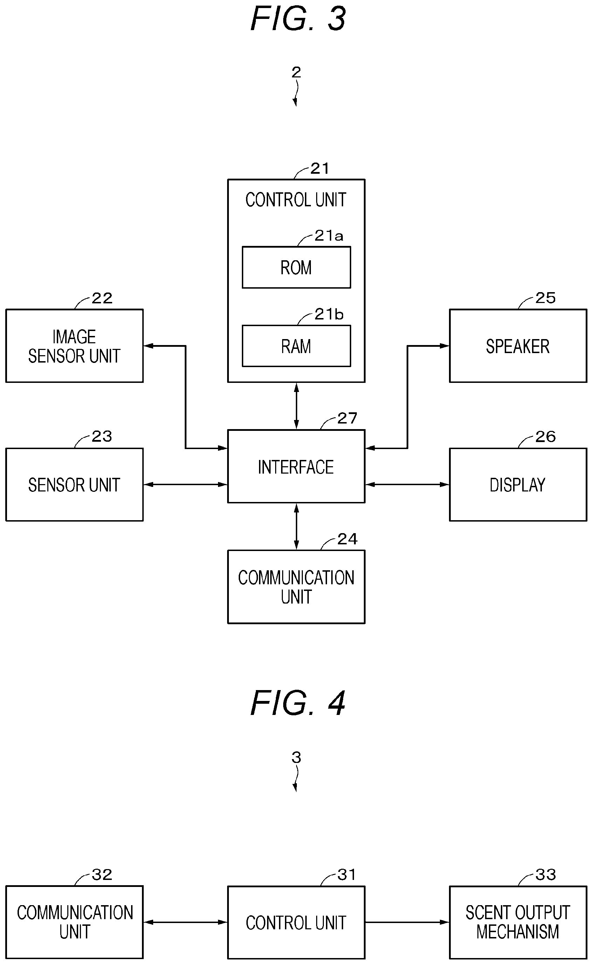

Fig. 3 is a block diagram illustrating a configuration example of the control device 2. The control device 2 has, for example, a control unit 21, an image sensor unit 22, a sensor unit 23, a communication unit 24, a speaker 25, and a display 26, and each of these units is connected via an interface 27 that is predetermined.

The control unit 21 includes a central processing unit (CPU), or the like, and controls each unit of the control device 2. The control unit 21 has a read only memory (ROM) 21a in which a program is stored, and a random access memory (RAM) 21b used as a work memory when the program is executed.

The control unit 21 recognizes a hand position or hand motion of the user by performing predetermined image processing on image data obtained by the image sensor unit 22. The control unit 21 outputs a control signal S1, which is information that controls output of scent, according to the hand position, the hand motion, or the like. The control unit 21 has a processing circuit (not illustrated) for performing image processing.

The image sensor unit 22 is configured by a charge coupled device (CCD), a complementary metal oxide semiconductor (CMOS), or the like. The image sensor unit 22 photoelectrically converts object light that enters via a lens unit (not illustrated) into a charge amount (image data), and outputs the image data. The image data is supplied to the control unit 21 via the interface 27.

The sensor unit 23 is a general term for configuration other than the image sensor unit 22. As the sensor unit 23, a motion sensor, specifically, an acceleration sensor, a gyro sensor, an electronic compass, a barometric pressure sensor, or the like, is exemplified. The sensor unit 23 may have a biosensor that measures biological information (for example, blood pressure, a pulse, body temperature, or the like) of a user of the control device 2. Furthermore, the sensor unit 23 may have a pressure sensor for detecting whether or not the user wears the control device 2, a microphone that detects sound, or the like. The configuration of the sensor unit 23 can be changed as appropriate according to content of control performed by the control device 2.

The communication unit 24 is for performing wireless communication with the scent output device 3, for example. The communication unit 24 has a modem circuit according to a communication method, an antenna, or the like. Examples of the wireless communication include local area network (LAN), Bluetooth (registered trademark), Wi-Fi (registered trademark), or wireless USB (WUSB), and the like.

The speaker 25 outputs sound. The speaker 25 emits predetermined sound according to control of the control unit 21. The sound may be any sound such as human voice or natural sound. Sound source may be data stored in the control unit 21 or may be data acquired via the communication unit 24.

The configuration of display 26 corresponds to that of the above-described right image display unit 6a and left image display unit 6b. The display 26 includes a liquid crystal display (liquid crystal LCD), an organic light emitting diode (OLED), or the like.

### [About scent output device]

### (Configuration example)

Fig. 4 is a block diagram illustrating a main configuration example of the scent output device 3. The scent output device 3 has, for example, a control unit 31, a communication unit 32, and a scent output mechanism 33.

The control unit 31 includes a CPU, or the like, and controls each unit of the scent output device 3. The control unit 31 has a ROM in which a program is stored, and a RAM used as a work memory when the program is executed. Note that illustrations of these are omitted. The control unit 31 performs control regarding emission of scent, or the like, on the basis of control signal S1 transmitted from the control device 2.

The communication unit 32 is for performing wireless communication with the control device 2, for example. The communication unit 32 has a modem circuit according to a communication method, an antenna, or the like.

The scent output mechanism 33 is a mechanism that actually emits scent, or the like. The scent output mechanism 33 emits scent by, for example, volatilizing source of liquid scent. Note that a known mechanism that emits scent can be applied to the scent output mechanism 33.

### (Specific examples)

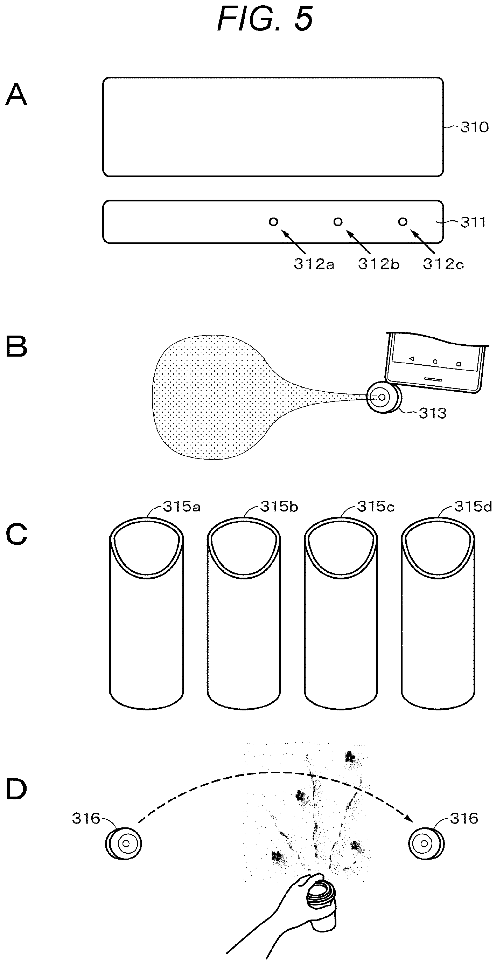

Specific examples of the scent output device 3 will be described with reference to Figs. 5A to 5D. A scent output device 3 exemplified in Fig. 5A has, for example, a housing 310, which is substantially rectangular and box-shaped in a top view. In the housing 310, source of scent is contained. A plurality of holes is provided on a side surface 311 in a longitudinal direction of the housing 310. In the example illustrated in Fig. 5A, three holes (holes 312a, 312b, and 312c) are provided. Note that, in a case where there is no need to distinguish the individual holes, the holes are referred to as a hole 312, as appropriate. The hole 312 is, for example, configured to be openable and closable.

Scent is released from inside of the housing 310 via the hole 312. For example, the scent is released from the inside of the housing 310 by opening the hole 312. Furthermore, release of the scent is stopped by closing the hole 312.

Direction in which the scent comes may be controlled by setting a hole 312 to be opened among the plurality of holes 312 as appropriate. For example, in a case where the user is present at a position facing a substantially center of the side surface 311, it is possible to perform control to cause the user to feel as if the scent comes from a right direction by opening only the hole 312c.

A degree of opening and closing of a hole 312 may be changed. By causing the hole 312 to open to a greater extent, intensity of scent can be increased. Furthermore, by narrowing the hole 312, the intensity of the scent can be reduced. Furthermore, a ventilation mechanism such as a fan may be provided in the housing 310. It is also possible to change the intensity of the scent according to intensity of the ventilation.

The example illustrated in Fig. 5B is an example of a scent output device 3 that can be attached to a portable apparatus (for example, the control device 2 according to the embodiments or a smartphone). The scent output device 3 illustrated in Fig. 5B has, for example, a housing 313 that is small and cylindrical. The housing 313 is attached to a housing of a smartphone or the like by an attachment mechanism (for example, a clip-shaped attachment mechanism) not illustrated. Then, scent is released from a predetermined hole of the housing 313. Needless to say, in this example also, intensity of the scent, or the like, can be changed. Note that, in Fig. 5B, the scent is schematically illustrated by a dotted hatching.

The example illustrated in Fig. 5C is an example of a scent output device 3 having a tubular housing. In Fig. 5C, four scent output devices 3 are illustrated, and each of the scent output devices 3 has a tubular housing 315a, 315b, 315c, or 315d. For example, scent is emitted from an upper part of a tubular housing. These scent output devices 3 are arranged at appropriate positions, and a scent output device 3 that emits scent is selected as appropriate. Therefore, control to emit scent from an arbitrary location becomes possible. Needless to say, the number of the scent output devices 3 is not limited to four, but an arbitrary number can be set.

The example illustrated in Fig. 5D is an example of a scent output device 3 that is movable. The scent output device 3 illustrated in Fig. 5D has a housing 316 that is small and cylindrical. Scent is released from the housing 316. The housing 316 is supported by, for example, a linear motor (not illustrated), or the like. The housing 316 is movable by driving the linear motor as appropriate, as schematically illustrated in Fig. 5D. By setting the housing 316 at an arbitrary position and allowing the scent to be released from the housing 316 at the position, the scent can be emitted from an appropriate position. That is, in the embodiments, intensity of scent, a timing at which emission of scent is stopped, or the like, can be arbitrarily set, in addition to a scent emission part.

### [About recognition regarding shape of hand]

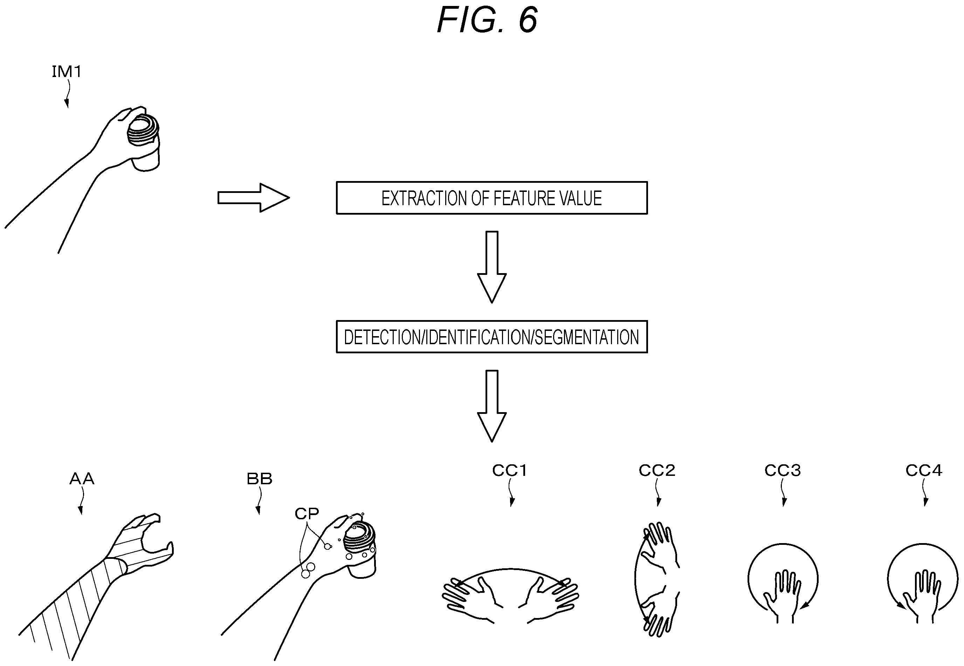

Next, a recognition method regarding a hand performed by the control unit 21 of the control device 2 will be described. Fig. 6 generalizes the recognition method. For example, an image (image IM1) of grabbing a coffee cup by hand is input. A feature value is calculated on the basis of the image IM1. Examples of the feature value include a feature value based on a convolutional neural network (CNN), a histograms of oriented gradients (HOG) feature value, and a feature value based on a scale invariant feature transform (SIFT).

Then, a hand position, or the like, is determined by analyzing an obtained feature value by a predetermined algorithm such as detection, identification, and segmentation. As an algorithm, the above-described CNN, boosting (Boosting), support vector machine (SVM), graph cut, or the like, can be applied.

Then, according to an analysis result, a hand position can be detected as indicated by a reference sign AA in Fig. 6. Note that the "hand" in the embodiments means a part further down a wrist, but may include an arm area. The "hand" may be a part (for example, only a finger), not an entire part further down the wrist. Furthermore, a hand position or pose can be detected as indicated by a reference sign BB in Fig. 6. Note that, in the drawing indicated by the reference sign BB, circles provided to the hand indicate characteristic points CP.

Moreover, by performing the above-described processing on images of a plurality of frames, it is also possible for the control device 2 to recognize hand motions, as indicated by reference signs CC (CC1 to CC4) in Fig. 6. The hand motion provided with a reference sign CC1 indicates an example of moving a hand in a horizontal direction (Horizontal). The hand motion provided with a reference sign CC2 indicates an example of moving a hand in a vertical direction (Vertical). The hand motion provided with a reference sign CC3 indicates an example of moving a hand clockwise (Clockwise). The hand motion provided with a reference sign CC4 indicates an example of moving a hand counterclockwise (Counterclockwise). Needless to say, a hand motion to be recognized is not limited to the exemplified ones, and various hand motions can be recognized. Furthermore, the above-described processing is not based on the exemplified algorithms, and a known method can be applied.

### (Examples of hand position estimation method)

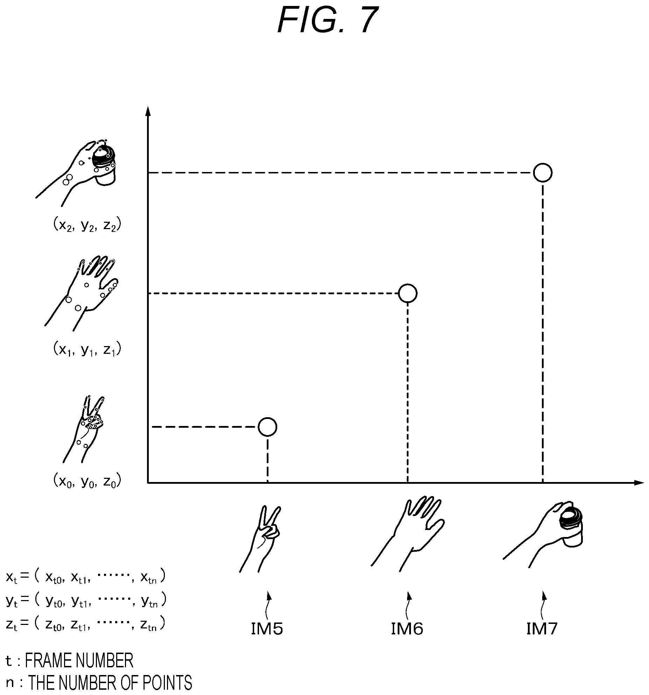

Next, an example of a method for estimating a hand position will be described with reference to Figs. 7 to 9. The horizontal axis of the graph illustrated in Fig. 7 indicates examples of an input image. In the examples illustrated in Fig. 7, three input images IM5, IM6, and IM7 are illustrated. The vertical axis of the graph illustrated in Fig. 7 is coordinates (position) of characteristic points of a hand corresponding to the input images. The coordinates are indicated as (xₜ, yₜ, zₜ) (where t is a frame number) . Furthermore, for example, xₜ is represented by

Xₜ = (Xₜ₀, xₜ₁... xₜₙ) (where n is the number of characteristic points). That is, xₜ indicates a set of x coordinate of each characteristic point. It is similar for yₜ and zₜ of each characteristic point.

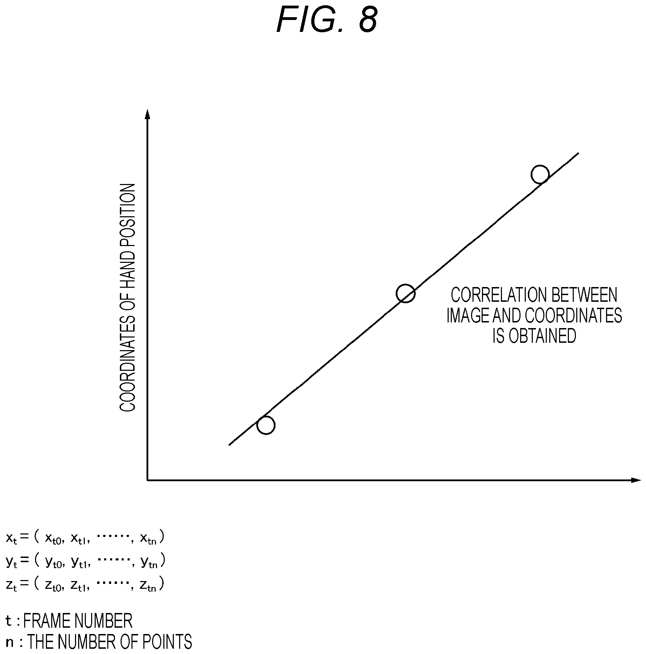

By learning coordinates for an input image, correlation between the input image and coordinates of the hand position is obtained as illustrated in Fig. 8. Although a linear correlation is illustrated in the example illustrated in Fig. 8, another correlation, such as a nonlinear correlation, may be used.

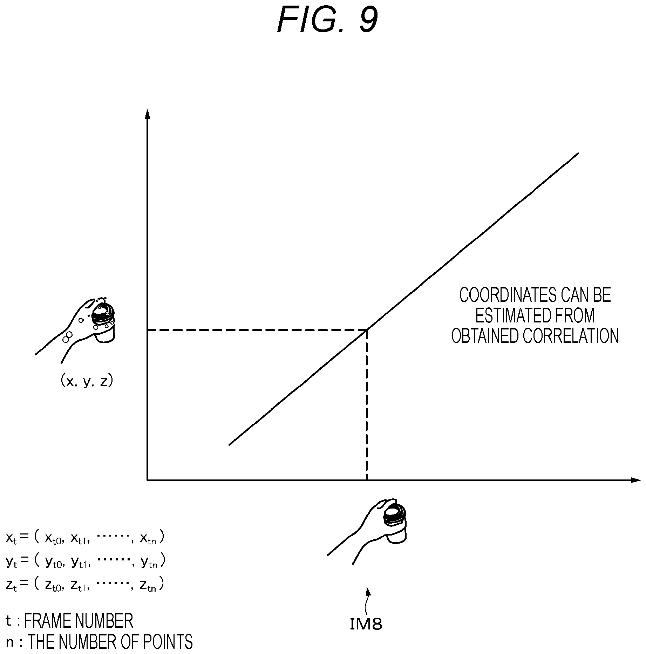

After correlation is obtained, for example, in a case where an image IM8 similar to the image IM7 is input as illustrated in Fig. 9, a coordinate position of the hand of the image IM8 can be estimated on the basis of the correlation. A hand position may be estimated by using the method described above.

### <First embodiment>

A first embodiment will be described. The first embodiment is an example in which a control unit 21 of a control device 2 identifies a hand position on the basis of a result of recognizing a hand, and outputs information for controlling output of scent from the position. Note that the "position" in this specification may be a position that exactly matches the position, or may be a position close to the position. A specific example of the closeness is a position closest to a target position (for example, a hand position or an object position) among a plurality of positions capable of control regarding output of scent.

### (Overview of processing)

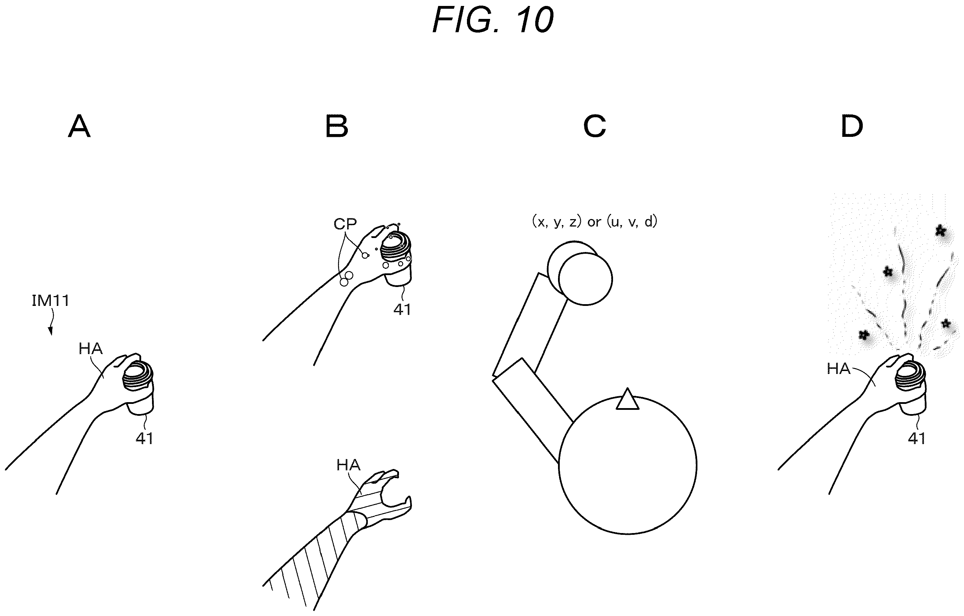

Figs. 10A to 10D are diagrams conceptually illustrating content corresponding to the first embodiment. For example, an image IM11 from a viewpoint of a user is input via an image sensor unit 22 of the control device 2. The Image IM11 is an image in which a user of the control device 2 grabs a coffee cup 41 by hand HA. Note that the coffee cup 41 may be a real object or may be a virtual object superimposed on real space. The image IM11 is supplied from the image sensor unit 22 to the control unit 21.

On the basis of the image IM11, the control unit 21 separates and detects the hand HA by using a characteristic point CP in the image IM11 as illustrated in Fig. 10B, pattern matching, or another method. Then, it is determined whether or not the detected hand HA has an object by using the position of the characteristic point CP, pattern matching, or another method. Note that, on the basis of sensing data of another sensor device (for example, a pressure sensor provided to the coffee cup 41), the hand HA may be detected, and whether the hand HA has the coffee cup 41 may be determined.

Then, as schematically illustrated in Fig. 10C, in a case where it is determined that the hand HA has an object, a three-dimensional position of the hand HA detected is calculated. The three-dimensional position may be three-dimensional coordinates (x, y, z) of the world coordinate system, or may be (u, v, d) of two-dimensional coordinates of a point projected on an image plane with depth information of the point included. For example, the three-dimensional position of the hand HA is obtained by the Hand pose detection technology. Furthermore, the three-dimensional position may be obtained by using a center of gravity of an area of the hand HA and depth information (depth information) of the part, which are obtained by the Hand Segmentation technology.

Then, as schematically illustrated in Fig. 10D, control is performed to emit scent from a location corresponding to the obtained three-dimensional position of the hand HA. In this embodiment, the scent is scent of coffee. An example of control to emit scent will be described. For example, it is assumed that a plurality of scent output devices 3 is arranged in space where the hand HA of the user is present. The control unit 21 generates a control signal S1 for a scent output device 3, among the plurality of scent output devices 3, arranged at a three-dimensional position (or a position closest to the position) of the hand HA. That is, the control signal S1 includes an identifier that identifies the scent output device 3 and data giving an instruction on emission of scent. This control signal S1 is simultaneously transmitted to each of the scent output devices 3 via a communication unit 204.

Each of the scent output devices 3 receives the control signal S1 via the communication unit 32. Then, a control unit 31 of each of the scent output devices 3 interprets the control signal S1, and interprets whether or not the control signal S1 is a signal addressed to the control unit 31. In a case where the control signal S1 is addressed to the control unit 31, the control unit 31 drives a scent output mechanism 33 and causes the scent output mechanism 33 to emit scent. With this arrangement, scent is emitted from a three-dimensional position of the hand HA, and the user can perceive that the scent is emitted from a visually recognized predetermined position.

Note that data for controlling intensity of scent may be included in the control signal S1 according to a position in a depth direction of an object (in this example, the coffee cup 41) that emits scent. For example, in a case where a distance in a depth direction to the user is long, the scent is caused to be weak, and in a case where the distance in the depth direction to the user is short, the scent is caused to be strong.

Furthermore, a plurality of scent output devices 3 may not be required. For example, in a case where a scent output device 3 is movable, the scent output device 3 may be moved to a position corresponding to a three-dimensional position of the hand HA, and scent may be caused to be emitted from the scent output device 3 after the movement.

### (Flow of processing)

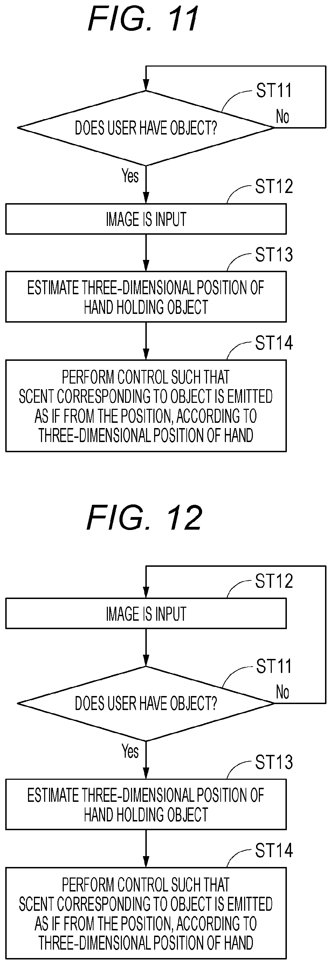

Fig. 11 is a flowchart illustrating a flow of processing according to the first embodiment. The processing described below is performed by, for example, the control unit 21. In step ST11, it is determined whether or not the user has an object (which may be a virtual object or a real object). The processing in step ST11 is performed, for example, as below. Output of a pressure sensor provided on the object changes according to gripping operation of the user, and a detection result is performed to the control device 2 by communication. The control unit 21 of the control device 2 can determine whether or not the user has an object on the basis of information supplied by this communication.

In a case where it is determined that the user does not have an object, the processing returns to step ST11, and determination processing in step ST11 is repeated. In a case where it is determined that the user has an object, the processing proceeds to step ST12.

In step ST12, an image data is supplied from the image sensor unit 22 to the control unit 21 via an interface 27. Then, the processing proceeds to step ST13.

In step ST13, from the image data, the control unit 21 estimates a three-dimensional position of the hand HA holding the object. As an estimation method, a known method other than the method described above can be applied. Then, the processing proceeds to step ST14.

In step ST14, according to the three-dimensional position of the hand HA, which is obtained in step ST13, the control unit 21 performs control such that scent corresponding to the object is released as if from the position. That is, the control unit 21 generates a control signal S1 for performing such control, and outputs the generated control signal S1. The control signal S1 is supplied to the scent output device 3, and a predetermined scent output device 3 performs operation of emitting the scent.

Note that the flow of the processing illustrated in Fig. 11 can be modified as in the flowchart illustrated in Fig. 12. That is, the processing in step ST12 may be performed before step ST11. In step ST12, an image data is supplied from the image sensor unit 22 to the control unit 21 via the interface 27. The control unit 21 determines whether or not the hand HA has an object on the basis of the image data. The control unit 21 determines, for example, a shape of the hand HA grabbing the object or distance between the hand HA and the object, or the like, on the basis of the image data, and determines whether or not the hand HA has the object. Because other processing is the same as the above-described processing, duplicated description will be omitted.

As described above, in the first embodiment, it is possible to perform processing to identify a position of a hand HA on the basis of a result of recognizing the hand HA and controlling output of a predetermined scent from the position.

### <Second embodiment>

Next, a second embodiment will be described. The second embodiment is an example in which a control unit 21 outputs information that controls output of scent on the basis of a result of recognizing a hand motion (gesture). Note that, unless otherwise specified, the items described in the first embodiment can be applied to the second embodiment.

### [Overview of processing]

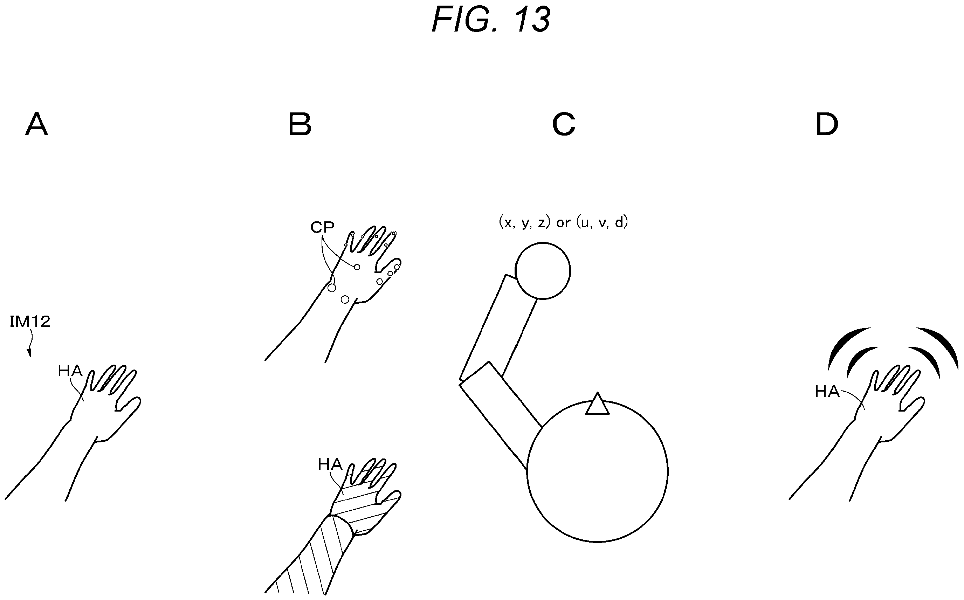

Figs. 13A to 13D are diagrams conceptually illustrating content corresponding to the second embodiment. Figs. 13A to 13C are similar to the items described in the first embodiment, and therefore will be described only schematically. That is, a three-dimensional position of a hand HA is detected on the basis of an input image IM12. In this embodiment, moreover, motion of the hand HA is further recognized. For example, a change in a characteristic point, a change in a shape of the hand HA, or the like, is detected, and a hand motion associated with the change is recognized as a predetermined motion of the hand HA. The recognition of the motion of the hand HA may be another known method (such as a method based on technology called hand gesture recognition). Then, output of scent is controlled on the basis of the three-dimensional position of the hand HA. At that time, control according to the recognized hand motion is performed. Note that when discriminating a type of scent, the type of the scent may be discriminated according to the motion of the hand HA and the recognized object.

### [Flow of processing]

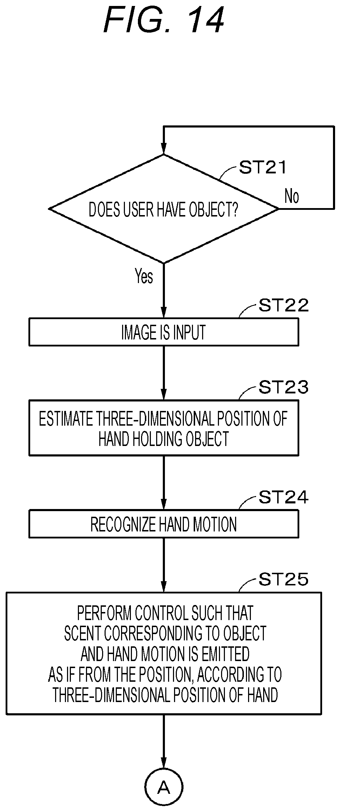

Fig. 14 is a flowchart illustrating a flow of processing according to the second embodiment. The processing described below is performed by, for example, the control unit 21. Because the processing in steps ST21 to ST23 is similar to the processing in steps ST11 to ST13 in the above-described first embodiment, duplicated description will be omitted. After step ST23, the processing proceeds to step ST24.

In step ST24, in a case where there is a motion of the hand HA, the control unit 21 recognizes the motion. Then, the processing proceeds to step ST25.

In step ST25, according to the three-dimensional position of the hand HA, the control unit 21 performs control such that scent corresponding to the object and the motion of the hand HA is emitted as if from the position.

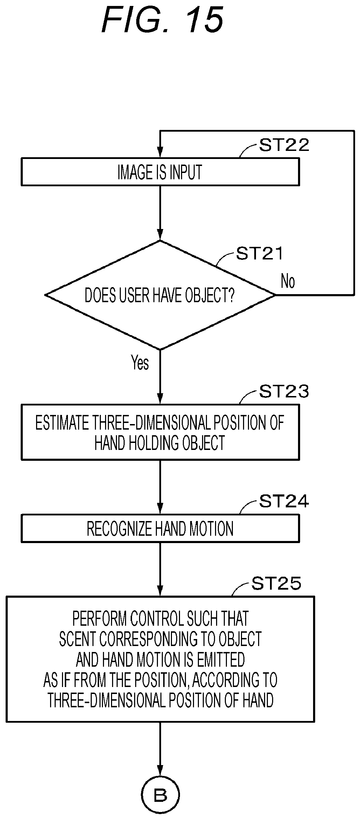

Note that the flow of the processing illustrated in Fig. 14 can be modified as in the flowchart illustrated in Fig. 15. That is, the processing in step ST22 may be performed before step ST21. In step ST22, an image data is supplied from an image sensor unit 22 to the control unit 21 via the interface 27. The control unit 21 determines whether or not the hand HA has an object on the basis of the image data. Because other processing is the same as the above-described processing, duplicated description will be omitted.

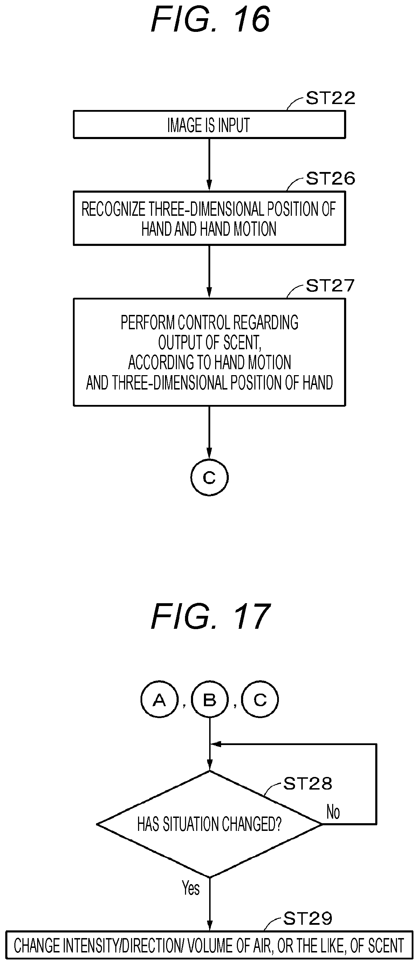

Moreover, as in the flowchart illustrated in Fig. 16, an object may not necessarily be present, and it may not be necessary to perform determination of whether or not a user has the object. That is, the processing in step ST22 is performed first, and an image data is input from the image sensor unit 22 to the control unit 21. Then, the processing proceeds to step ST26.

In step ST26, the control unit 21 recognizes the three-dimensional position of the hand HA and the motion of the hand HA on the basis of the image data input in step ST22. Then, the processing proceeds to step ST27.

In step ST27, the control unit 21 performs control regarding output of scent according to the three-dimensional position of the hand HA and the motion of the hand HA, which are recognized in step ST26.

Note that, not only control regarding output of scent but also control to cause a user to perceive a change in surrounding environment may be performed. For example, controlling emission of sound, emission of wind, or the like may be performed.

### [Modification of processing]

After the processing illustrated in Figs. 14 to 16 has been performed, the processing illustrated in the flowchart in Fig. 17 (the processing in step ST28 and step ST29) may be performed. Note that the circled letters "A", "B", and "C" illustrated in Figs. 14 to 17 are for indicating continuity of processing, and not for indicating specific processing.

In step ST28, the control unit 21 determines whether or not situation has changed. The change in the situation means, for example, a case where a new motion of the hand HA is detected or a case where the three-dimensional position of the hand HA changes. In a case where the situation does not change, the processing returns to step ST28, and determination processing in step ST28 is repeated. In a case where the situation has changed, the processing proceeds to step ST29.

In step ST29, control regarding output of scent according to the change in the situation is performed. For example, control is performed to change intensity of the scent and a direction in which the scent comes. Along with the control regarding output of the scent, control content other than the control may be changed. For example, volume of air output from a ventilation device, which is one of environmental change emission devices, may change according to a change in the situation.

### [Specific examples]

Specific examples corresponding to the second embodiment will be described. Table 1 below is a table illustrating specific examples corresponding to the second embodiment.

**[Table 1]**

| Pattern | Motion | Another input example | Output example |
|---|---|---|---|
| P1 | Touching a flower | | Scent of a flower is emitted from a position of a hand |
| P2 | Crushing (one hand/both hands) a fruit | | Scent of a fruit is emitted from a position of a hand |
| P3 | Rocking a glass | | Scent of wine is emitted from a position of a hand |
| P4 | - Bloom gesture | Chanting a magic spell (Not necessary) | Scent that matches magic given off from a position of a hand is emitted (Wind may be output) (Sound may be output) |
| | - Raising an index finger | | |
| P5 | Gripping an object such as a game controller | Gripping an object hard) | Intensity of scent is changed according to gripping force |
| P6 | Applause | Recognizing sound of applause also (Not necessary) | Intensity of scent is changed according to volume of applause from a degree of opening of a hand or the like (Wind may be output) (Sound may be output) |
| P7 | Stroking a flower and moving a hand closer | | Scent of a flower is emitted from a position of a hand |

Table 1 illustrates each of pattern numbers, recognized motions of the hand HA (there may be a case where an object is included), examples of input from a sensor device different from the image sensor unit 22, and scent output examples.

In a case of Pattern P1, a "flower" is recognized as an object, "touch" motion recognition is recognized as a motion of the hand HA, and, from these results, the motion of the hand HA "touching a flower" is recognized. Note that, in this case, the flower may be a real object that has been recognized as a flower by image recognition, or the like, or may be a virtual object that has already been recognized as a flower. In this case, control is performed to emit scent of a flower from a position corresponding to a three-dimensional position of the hand HA. Then, a scent output device 3 operates to emit scent of the flower.

In a case of Pattern P2, a "fruit" is recognized as an object, a "crushing" motion is recognized as a motion of the hand HA, and, from these results, the motion of the hand HA "crushing a fruit" is recognized. The hand HA may be one hand or may be both hands. Note that, in this case, the fruit may be a real object that has been recognized as a fruit by image recognition, or the like, or may be a virtual object that has already been recognized as a fruit. In this case, control is performed to emit scent of fruit from a position corresponding to a three-dimensional position of the hand HA. Then, the scent output device 3 operates to emit scent of the fruit.

In a case of Pattern P3, a "wine glass" is recognized as an object, and a turning motion is recognized as a motion of the hand HA. From these results, the motion of a hand HA "rocking a wine glass" is recognized. Note that, in this case, the wine glass may be a real object that has been recognized as a wine glass by image recognition, or the like, or may be a virtual object that has already been recognized as a wine glass. In this case, control is performed to emit scent of wine from a three-dimensional position of the hand HA. Then, the scent output device 3 operates to emit scent of the wine.

Pattern P4 is a pattern where no object is present. As a motion of the hand HA, for example, a motion of the hand HA as if when using magic is recognized. Such motion of the hand HA includes a bloom gesture (motion to open the hand HA clenched near a chest) and a motion to raise an index finger. In a case where such motion of the hand HA is recognized, scent suitable for the magic is released from a three-dimensional position of the hand HA (in a case of the bloom gesture, a three-dimensional position of when the hand HA is opened). The scent output device 3, in a case of magic dealing with a flame for example, operates to emit burnt scent from a three-dimensional position of the hand HA. Thus, in a case of controlling output of scent according to a motion of the hand HA, presence of an object is not necessarily required.

Note that, when a motion of the hand HA is recognized, another input may be referred to in order to recognize a meaning of the motion of the hand HA. In a case of Pattern P4, another input is sound. For example, the scent output device 3 may emit burnt scent along with the motion of the hand HA in a case where voice chanting a magic spell is detected. Voice can be detected by a sensor unit 23, for example. By referring to another input, more appropriate control can be performed, and inappropriate control due to erroneous recognition can be prevented.

Furthermore, in order to give a more realistic feeling, control regarding, not only output of scent, output of wind or output or sound (for example, sound of a flame that instantaneously gives off) by the environmental change emission device may be performed.

Pattern P5 is an example of scent control associated with a change in situation. In Pattern P5, for example, a game controller is recognized as an object, and a motion to grip the object is detected as a motion of the hand HA. Here, it is assumed that gripping force is increased. Change in the gripping force can be detected by the sensor unit 23, which is a pressure sensor, as a specific example. In a case where the gripping force increases, intensity of the scent changes. For example, in a case where the gripping force is increased, control to increase the intensity of the scent is performed.

In a case of Pattern P6, a motion to "applaud" is recognized as a motion of the hand HA. Volume of the applause can be recognized from a degree of opening of the hand HA (a distance between both hands) at a time of the applause. Control to change intensity of scent may be performed according to a degree of the applause. Note that, in a case of Pattern P6, together with the motion of the hand HA, sound of the applause may be recognized by sound recognition. Control regarding, not only output of scent, output of wind (for example, emission of wind from a part touched by both hands) or output of sound by the environmental change emission device may be performed.

Pattern P7 is an example of scent control based on a continuous motion of the hand HA. In Pattern P7, for example, a "flower" is recognized as an object. Note that, in this case, the flower may be a real object that has been recognized as a flower by image recognition, or the like, or may be a virtual object that has already been recognized as a flower. Then, as a motion of the hand HA, a motion to move the hand HA, for example, close to a nose after stroking the flower is recognized. In such a case, control is performed to emit flower scent from a three-dimensional position of the hand HA moved near the nose.

A detection result by a temperature sensor may be used as another input. For example, in a case where "meat" is recognized as an object and a motion to "grill meat" is recognized as a motion of the hand HA, according to a change in temperature, control to output sound of grilling meat may be performed along with control to emit scent of grilling meat.

### <Third embodiment>

Next, a third embodiment will be described. The third embodiment is an example in which a control unit 21 outputs information that cancels scent on the basis of a recognition result regarding a motion of a hand HA (gesture). Note that, unless otherwise specified, the items described in the first and second embodiments can be applied to the third embodiment. Note that, not on the basis of a motion of the hand HA, but on the basis of a shape of the hand HA at a predetermined position (for example, a shape of a spread hand near a nose, as if blocking the nose), information that cancels the scent may be output.

### [Overview of processing]

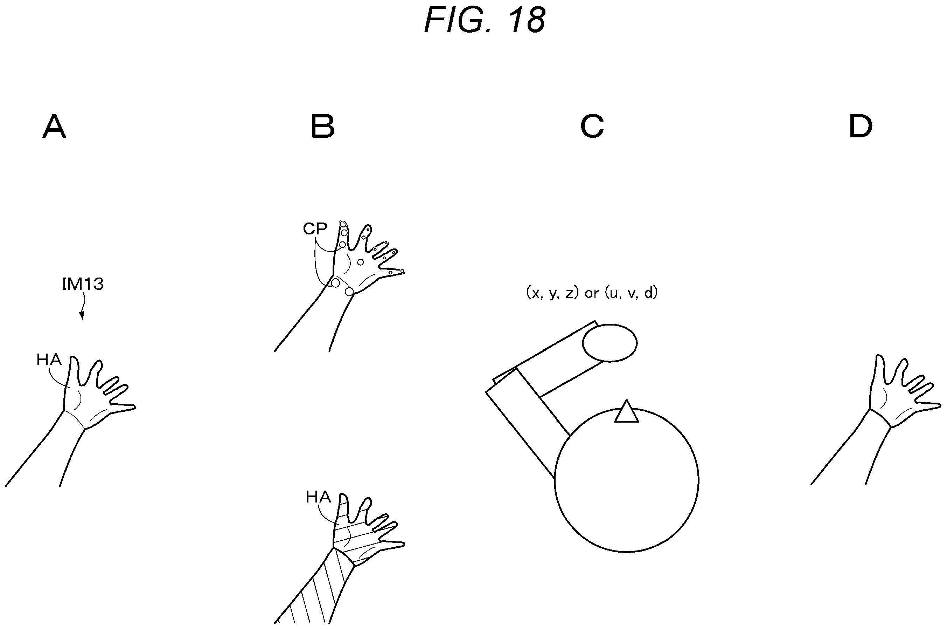

Figs. 18A to 18D are diagrams conceptually illustrating content corresponding to the third embodiment. Figs. 18A to 18C are similar to the items described in the first embodiment, and therefore will be described only schematically. That is, a three-dimensional position of the hand HA is detected on the basis of an input image IM13. In this embodiment, for example, at least one of the shape of the hand HA or the hand HA (hereinafter, referred to as a motion of a hand HA, or the like, as appropriate) is detected according to a position of or a change in a characteristic point, a change in the shape of the hand HA, or the like. In a case where the detected motion of the hand HA, or the like, is a preset motion, or the like, the control unit 21 determines that a user has an aversion to the scent, and outputs, as a control signal S1, information that cancels the scent. Note that, in this embodiment, canceling scent may mean weakening intensity of the scent, may mean causing emission of the scent to be stopped, or may mean controlling ventilation, or the like, to immediately eliminate the scent (hereinafter, referred to as deodorization as appropriate), along with causing the emission of the scent to be stopped.

### [Flow of processing]

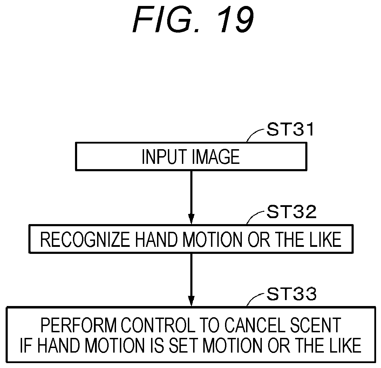

Fig. 19 is a flowchart illustrating a flow of processing according to the third embodiment. The processing described below is performed by, for example, the control unit 21. In step ST31, an image data is input from the image sensor unit 22 to the control unit 21 via an interface 27. Then, the processing proceeds to step ST32.

In step ST32, the control unit 21 recognizes a hand motion, or the like. Then, the processing proceeds to step ST33.

In step ST33, the control unit 21 executes control to cancel the scent if the hand motion, or the like, is a set motion, or the like. That is, the control unit 21 generates a control signal S1 for canceling the scent, and supplies the generated control signal S1 to a scent output device 3. The scent output device 3 operates to cancel the scent on the basis of the control signal S1. Note that, when performing control to deodorize the scent, the control unit 21 generates a control signal S2 and supplies the generated control signal S2 to an environmental change emission device. The environmental change emission device performs processing for deodorizing the scent by performing ventilation, or the like.

### [Specific examples]

Specific examples corresponding to the third embodiment will be described. Table 2 below is a table illustrating specific examples corresponding to the third embodiment.

**[Table 2]**

| Pattern | System of motion of hand | Another input | Output |
|---|---|---|---|
| P8 | Holding out a spread hand in front of eyes | Voice ("Stinky!", "Cut it out!", "Stop!", "End!", or the like) sniffling, coughing, or the like | Stop output of scent, soften scent, deodorize scent |
| P9 | Holding a hand over an object (one hand/both hands) | Voice ("Stinky!", "Cut it out!", "Stop!", "End!", or the like) sniffling, coughing, or the like | Stop scent of an object, soften scent of an object, deodorize scent |
| P10 | Concealing an object by one hand/both hands | Voice ("Stinky!", "Cut it out!", "Stop!", "End!", or the like) sniffling, coughing, or the like | Stop scent of an object, soften scent of an object, deodorize scent |

In Pattern P8 in Table 2, as a motion of the hand HA, or the like, a motion to hold out a hand HA spread in front of eyes (more specifically, around in front of a nose) is recognized. This operation is recognized as operation showing that the user has an aversion to the smell. Therefore, the control unit 21 generates a control signal S1 for canceling the scent, and supplies the generated control signal S1 to the scent output device 3. The scent output device 3 operates according to the control signal S1, and, for example, stops output of the scent, softens the scent (reduces the scent), or performs deodorization.

Note that the above-described control may be performed taking another input into account. For example, in a case where, along with the above-described motion of the hand HA, specific voice (for example, "stinky", "cut it out", "stop", "end", or the like) or specific sound (for example, sound of sniffling or coughing) by sound input is detected by a sensor unit 23, control to cancel the scent may be performed. Furthermore, control to cancel scent may be performed with reference to information based on a biological sensor, such as rough breathing or high body temperature, information based on a temperature sensor that a surrounding temperature increases due to burning, information based on a smell sensor, information based on a line-of-sight detection sensor, such as an unstable line-of-sight, or other information. In this manner, by referring to another input, more appropriate control can be performed, and inappropriate control due to erroneous recognition can be prevented.

Pattern P9 is an example in which a motion to hold the hand HA over an object is detected as a motion of hand HA. Pattern P10 is an example in which a motion to conceal an object by the hand HA is detected as a motion of hand HA. Note that, in Patterns P9 and P10, the object may be a real object or may be a virtual object. Furthermore, in Patterns P9 and P10, the hand HA may be one hand or may be both hands. Control to cancel the scent is performed similarly in a case of Patterns P9 and P10. Other than the exemplified patterns, for example, control may be performed to cancel scent in a case where, for example, a motion of the hand HA waving near a nose is detected, or the like. As described above, control to cancel scent can be performed according to a motion of the hand HA, or the like.

Note that, in a case of Patterns P9 and P10 also, the above-described control may be performed taking another input into account. For example, in a case where, along with the above-described motion of the hand HA, specific voice (for example, "stinky", "cut it out", "stop", "end", or the like) or specific sound (for example, sound of sniffling or coughing) by sound input is detected by a sensor unit 23, control to cancel the scent may be performed. Furthermore, control to cancel scent may be performed with reference to information based on a biological sensor, such as rough breathing or high body temperature, information based on a temperature sensor that a surrounding temperature increases due to burning, information based on a smell sensor, information based on a line-of-sight detection sensor, such as an unstable line-of-sight, or other information. In this manner, by referring to another input, more appropriate control can be performed, and inappropriate control due to erroneous recognition can be prevented.

### <Modifications>

Although the embodiments of the present disclosure have been specifically described above, the content of the present disclosure is not limited to the above-described embodiments, and various modifications based on the technical idea of the present disclosure are possible.

Although a hand has been described as an example in the above-described embodiments, the present disclosure can be applied to a part of a body such as a foot or an elbow. Furthermore, the control device is not limited to glasses-type wearable apparatus, and may be a wearable apparatus worn on a shoulder, a wristband-type wearable apparatus, a head-up display, or the like. Furthermore, the control device is not limited to a wearable apparatus.

In the above-described embodiments, even in a case where the object is a real object, the real object does not necessarily need to be an object that emits scent. Assuming that the real object emits scent, processing to control output of the scent from a position of the real object may be performed as described in the above-described embodiments.

Part of the processing by the control device in the above-described embodiments may be performed on the cloud. For example, processing executed by the control unit 21 may be performed by a server device, or the like, on the cloud.

In the above-described embodiments, control regarding output of scent in consideration of passage of time may be performed. For example, after control to emit scent is performed, control may be performed such that intensity of the scent gradually decreases with passage of time.

The present disclosure can be applied to various apparatuses. For example, the present disclosure can be applied not only to an amusement apparatus such as a game apparatus, but also to a simulation apparatus for medical care, cooking, disaster relief, or the like.

The present disclosure can also be actualized by a device, a method, a program, a system, or the like. For example, by enabling download of a program that performs a function described in an above-described embodiment and by a device, which does not have the function described in the embodiment, downloading and installing the program, control described in the embodiment can be performed in the device. The present disclosure can also be actualized by a server that distributes such a program. Furthermore, the items described in each of the embodiments and the modifications can be combined as appropriate.

The present disclosure may have the following configurations.
(1) A control device including
   a control unit that outputs information that controls output of scent on the basis of a result of recognizing a hand.
(2) The control device according to (1),
   in which the control unit identifies a hand position on the basis of a result of recognizing a hand, and outputs information for controlling output of scent from the position.
(3) The control device according to (2),
   in which the information includes information for controlling output of scent to a device closest to the hand position identified among a plurality of devices that emits scent.
(4) The control device according to (1),
   in which the control unit outputs information that controls output of scent on the basis of a result of recognizing a hand motion.
(5) The control device according to any one of (1) to (4),
   in which the information that controls output of scent includes at least one of information that causes scent to be emitted, information that causes intensity of scent to be changed, information that causes a direction in which scent comes to be changed, information that stops emission of scent, or information indicating time duration for which scent emission lasts.
(6) The control device according to any one of (1) to (5),
   in which the control unit outputs information for performing control to cause a user to perceive a change in surrounding environment along with the information that controls output of scent.
(7) The control device according to (6),
   in which the control to cause a user to perceive a change in surrounding environment includes at least one of control to reproduce sound, control to emit wind, or control to change surrounding brightness.
(8) The control device according to any one of (1) to (5),
   in which the control unit outputs information that controls output of scent on the basis of the result of recognizing a hand and a recognition result with respect to a predetermined input.
(9) The control device according to (8),
   in which the predetermined input includes input that is detectable by at least one of a sound sensor, a temperature sensor, or a biological sensor.
(10) The control device according to any one of (1) to (9),
   in which the control unit recognizes a hand on the basis of an image corresponding to a viewpoint of a user.
(11) The control device according to (10),
   in which the image includes an actual image or a virtual image.
(12) The control device according to any one of (1) to (11), further including
   a scent emission unit that performs output of scent based on the information.
(13) The control device according to any one of (1) to (12),
   the control device being configured as a wearable apparatus.
(14) A control method including
   outputting, by a control unit, information that controls output of scent on the basis of a result of recognizing a hand.
(15) A program that causes a computer to execute a control method including
   outputting, by a control unit, information that controls output of scent on the basis of a result of recognizing a hand.

### REFERENCE SIGNS LIST

- 1: Control system
- 2: Control device
- 3: Scent output device
- 21: Control unit
- 22: Image sensor unit
- 23: Sensor unit

## Claims

1. A control device comprising
a control unit that outputs information that controls output of scent on a basis of a result of recognizing a hand.

2. The control device according to claim 1,
wherein the control unit identifies a hand position on a basis of a result of recognizing a hand, and outputs information for controlling output of scent from the position.

3. The control device according to claim 2,
wherein the information includes information for controlling output of scent to a device closest to the hand position identified among a plurality of devices that emits scent.

4. The control device according to claim 1,
wherein the control unit outputs information that controls output of scent on a basis of a result of recognizing a hand motion.

5. The control device according to claim 1,
wherein the information that controls output of scent includes at least one of information that causes scent to be emitted, information that causes intensity of scent to be changed, information that causes a direction in which scent comes to be changed, information that stops emission of scent, or information indicating time duration for which scent emission lasts.

6. The control device according to claim 1,
wherein the control unit outputs information for performing control to cause a user to perceive a change in surrounding environment along with the information that controls output of scent.

7. The control device according to claim 6,
wherein the control to cause a user to perceive a change in surrounding environment includes at least one of control to reproduce sound, control to emit wind, or control to change surrounding brightness.

8. The control device according to claim 1,
wherein the control unit outputs information that controls output of scent on a basis of the result of recognizing a hand and a recognition result with respect to a predetermined input.

9. The control device according to claim 8,
wherein the predetermined input includes input that is detectable by at least one of a sound sensor, a temperature sensor, or a biological sensor.

10. The control device according to claim 1,
wherein the control unit recognizes a hand on a basis of an image corresponding to a viewpoint of a user.

11. The control device according to claim 10,
wherein the image includes an actual image or a virtual image.

12. The control device according to claim 1, further comprising
a scent emission unit that performs output of scent based on the information.

13. The control device according to claim 1,
the control device being configured as a wearable apparatus.

14. A control method comprising
outputting, by a control unit, information that controls output of scent on a basis of a result of recognizing a hand.

15. A program that causes a computer to execute a control method comprising
outputting, by a control unit, information that controls output of scent on a basis of a result of recognizing a hand.
